(19) Europäisches Patentamt
European Patent Office
Office européen des brevets

(11) Veröffentlichungsnummer: **0 191 386**
**B1**

# (12) EUROPÄISCHE PATENTSCHRIFT

(45) Veröffentlichungstag der Patentschrift: **18.10.89**

(51) Int. Cl.⁴: **C 07 C 127/00, C 08 G 18/38**

(21) Anmeldenummer: **86101318.3**

(22) Anmeldetag: **01.02.86**

## (54) Harnstoff- sowie Ester- oder Amidgruppen aufweisende Polyhydroxyverbindungen, Verfahren zu ihrer Herstellung und ihre Verwendung.

(30) Priorität: **13.02.85 DE 3504967**

(43) Veröffentlichungstag der Anmeldung: **20.08.86 Patentblatt 86/34**

(45) Bekanntmachung des Hinweises auf die Patenterteilung: **18.10.89 Patentblatt 89/42**

(84) Benannte Vertragsstaaten: **BE DE FR GB IT**

(56) Entgegenhaltungen: **Keine Entgegenhaltungen**

(73) Patentinhaber: **BAYER AG, D-5090 Leverkusen 1 Bayerwerk (DE)**

(72) Erfinder: **Ballé, Gerhard, Dr., Nietzsche-Strasse 4, D-5090 Leverkusen (DE)**
Erfinder: **Grögler, Gerhard, Dr., von-Diergardt-Strasse 48, D-5090 Leverkusen (DE)**
Erfinder: **Meckel, Walter, Dr., Zonserstrasse 9, D-4040 Neuss (DE)**

EP 0 191 386 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung sind Harnstoff- sowie Ester- oder Amidgruppen aufweisende Polyhydroxyverbindungen, Verfahren zu ihrer Herstellung und ihre Verwendung.

Die neuen Verbindungen werden durch Umsetzung von Carbamoyllactamen (aus Lactamen und Polyisocyanaten) mit mehrwertigen Alkoholen oder Monoamino-Alkoholen in Gegenwart katalytischer Mengen eines stark basischen Katalysators, z.B. eines Alkalialkoholats, bei erhöhten Temperaturen erhalten:

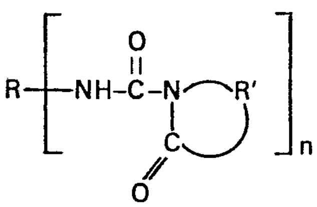

+ n HO–R″–$(OH)_a$

st. Base

erhöhte Temperatur (I)

oder

+ n $H_2N$.R‴–$(OH)_a$

st. Base

erhöhte Temperatur (II)

R Reste von Polyisocyanaten,
R′ Alkylenreste,
R″ und R‴ geradkettige oder verzweigte aliphatische oder cycloaliphatische Reste,

$n \geqslant 1$,

$a \geqslant 1$.

Die Harnstoff- sowie Ester- bzw. Amidgruppen aufweisenden Polyole der Formeln (I) bzw. (II)

$$R\text{–}[HN\cdot\overset{O}{C}\text{–}NH\text{–}R'\text{–}\overset{O}{C}\text{–}O\cdot R'\text{–}(OH)_a]_n \quad (I)$$

$$R\text{–}[HN\cdot\overset{O}{C}\text{–}NH\text{–}R'\text{–}\overset{O}{C}\text{–}NH\cdot R'''(OH)_a]_n \quad (II)$$

stellen kristalline, höherschmelzende Verbindungen dar, die als Reaktionskomponente zum Aufbau von PU-Elastomeren, insbesondere als feinverteilte Komponenten in Einkomponenten-Polyurethanreaktionsmischungen, verwendet werden.

Die Carbamoyllactame, d.h. die Addukte von Lactamen an Isocyanate, sind bekannte Verbindungen, die in der Polyurethan- und Polyamidchemie technisches Interesse gefunden haben. Die Addukte von Lactamen, insbesondere ε-Caprolactam, an Polyisocyanate oder an NCOfunktionelle Voraddukte von Polyisocyanaten mit Polyolen, Polyaminen oder Polyaminopolyolen, sind als verkappte, unter Lagerbedingungen nichtreaktive Isocyanate bekannt. Sie können mit NCO-reaktiven Stoffen, z.B. Polyolen, zu Einkomponentensystemen formuliert werden, die erst beim Erhitzen unter Rückspaltung des Lactamaddukts aushärten. Dabei kann als flüchtiges Spaltprodukt das Lactam und im hinterbleibenden Produkt die Urethangruppe nachgewiesen werden, z.B.

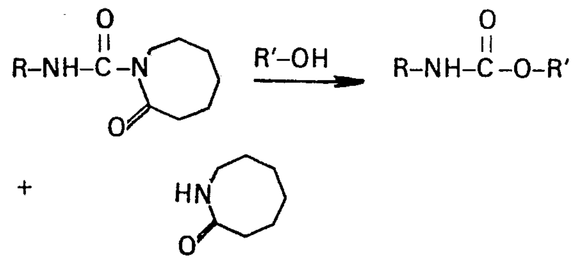


Eine technisch bedeutsame Rolle spielen die Acyllactame und Carbamoyllactame bei der aktivierten anionischen Lactampolymerisation, wo sie bekanntlich die Träger des Kettenstarts und des Kettenwachstums darstellen. Zum Mechanismus der anionischen Lactampolymerisation siehe E.H. Mottus, R.M. Hedrick, J.M. Butler, US Pat. 3 017 391 (Monsanto, 1962); ebenso H.K. Reinschüssel, «Lactams», in: Kinetics and Mechanisms of Polymerizations, Vol. 2, Ring Opening Polymerizations, Hrsg. K.C. Frisch, S. 303–326, M. Dekker, New York 1969.

Wir haben nun gefunden, dass es unter relativ milden Bedingungen möglich ist, den Carbamoyllactamring mit Alkoholen zu öffnen, wenn katalytische Mengen starker Basen anwesend sind. Diese Reaktion verläuft glatt und mit hoher Ausbeute schon bei relativ milden Bedingungen, z.B. in siedendem Methanol oder Ethanol. Die Nebenreaktion der Urethanbildung unter Lactamabspaltung wird dabei nicht beobachtet.

Man macht von diesem Prinzip der Isocyanatverkappung vor allem auf dem Lacksektor Gebrauch; die Temperaturen, die zur Auslösung der Rückspaltung nötig sind, liegen allerdings sehr hoch, im Falle des ε-Caprolactams oberhalb von 160 °C.

Acyllactame spielen eine Rolle in der Aminosäure- und Peptidchemie. Bekannt ist, dass Acyllactame den Lactamring hydrolytisch öffnen, wobei die Reaktionsbedingungen sehr sorgfältig gewählt und kontrolliert werden müssen, damit nicht anstelle von oder gleichzeitig mit der Ringöffnung die Abspaltung des Acylrestes erfolgt.

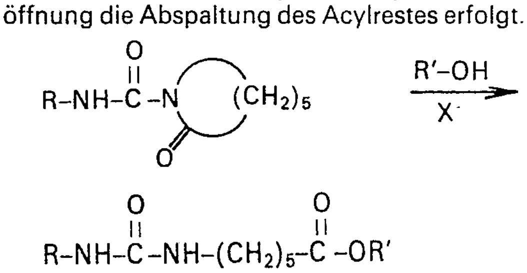


$X^-$ = Base, z.B. Alkoholatanion.

Verwendet man als Alkoholkomponente ein Polyol, etwa einen Alkohol, so gelangt man zu neuen Polyhydroxylverbindungen, die neben der Hydroxyl- und Estergruppe noch Harnstoffgruppierungen enthalten.

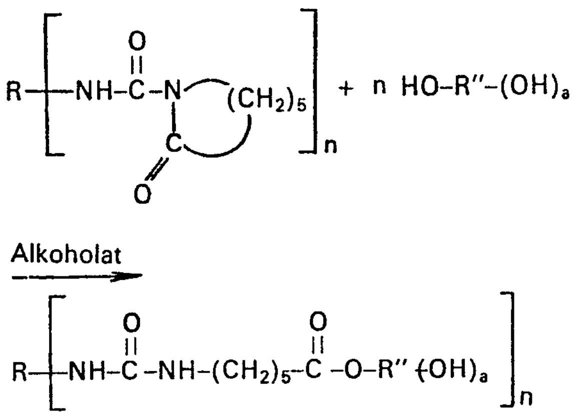


Hierin bedeuten R den Kohlenwasserstoff- oder heterocyclischen Rest eines Polyisocyanats der Funktionalität n und R'' den Rest eines mehrwertigen Alkohols der Funktionalität (a+1).

Ersetzt man das Glykol durch einen Aminoalkohol, so entstehen nicht Aminoester, sondern durch eine konzertierte oder nachfolgende Umlagerung die entsprechenden Hydroxyamide:

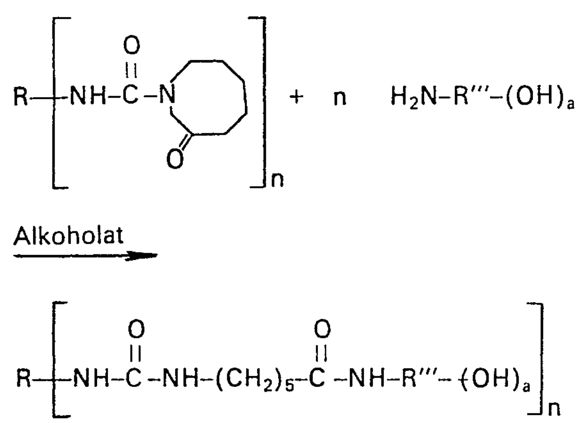


Die vorliegende Erfindung betrifft demgemäss Harnstoff- sowie Ester- bzw. Amidgruppen aufweisende Polyhydroxylverbindungen der allgemeinen Formeln (I) bzw. (II),

$$R-[HN\overset{O}{\overset{\|}{C}}-NH-R'-\overset{O}{\overset{\|}{C}}-O\cdot R'-(OH)_a]_n \qquad (I)$$

$$R-[HN\overset{O}{\overset{\|}{C}}-NH-R'-\overset{O}{\overset{\|}{C}}-NH\cdot R'''(OH)_a]_n \qquad (II)$$

worin

R der Rest eines aliphatischen, cycloaliphatischen, aromatischen oder heterocyclischen, gegebenenfalls modifizierten Polyisocyanats oder eines NCO-Voraddukts, insbesondere ein $C_2$ bis $C_{36}$-Alkylen-, $C_5$ bis $C_{15}$-Cycloalkylen-, $C_6$ bis $C_{15}$-Arylen- oder heterocyclischer Rest mit der Wertigkeit n,

R' ein $C_3$ bis $C_{11}$-Alkylen, bevorzugt $C_3$-, $C_4$-, $C_5$-, $C_{10}$- oder $C_{11}$-Alkylen-, insbesondere $C_5$-Alkylen-,

R'' ein Rest eines mehrwertigen Alkohols der Funktionalität (a+1),

R''' der Rest eines Monoaminoalkohols mit einer Hydroxylfunktionalität a) und

a eine ganze Zahl ≥ 1, vorzugsweise 1 bis 5; insbesondere 1 bis 3 und besonders bevorzugt 1 oder 2, und

n 2 bis 6, vorzugsweise 2 bis 3, ist.

Bevorzugt sind Polyhydroxylverbindungen der Formeln (I) und (II), worin

R einen Rest mit der Formel

$-(CH_2)_6-$, $-(CH_2)_6-N(CO-NH-(CH_2)_6-)-CO-NH-(CH_2)_6-$,

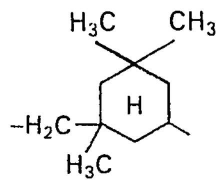 , 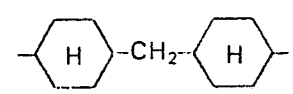 ,

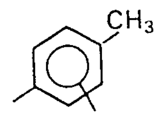 , 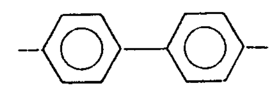 ,

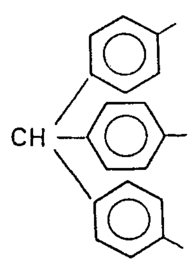 ,

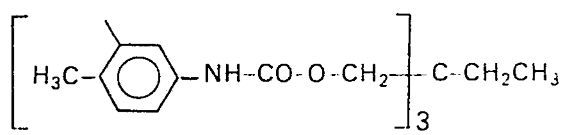

oder

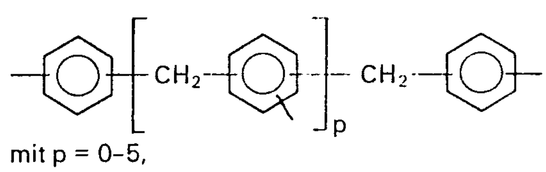

mit p = 0–5,

R' einen Rest der Formeln

$-(CH_2)_3-$, $-(CH_2)_4-$, $-(CH_2)_5-$ oder $-(CH_2)_{11}-$, insbesondere einen $-(CH_2)_5$-Rest;

R'' einen linearen oder verzweigten aliphatischen Rest oder cycloaliphatischen Rest mit (a+1) freien Valenzen und

R''' einen linearen oder verzweigten aliphatischen Rest oder einen cycloaliphatischen Rest mit (a+1) freien Valenzen, bedeutet.

Weiterer Erfindungsgegenstand ist ein Verfahren zur Herstellung der Polyhydroxylverbindungen (I) bzw. (II), dadurch gekennzeichnet, dass man Carbamoyllactamgruppen enthaltende Verbindungen der allgemeinen Formel (III)

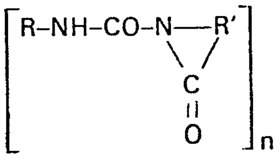 (III)

mit einem mehrwertigen Alkohol der Formel (IV) oder einem Monoaminoalkohol der Formel (V),

$$HO\text{-}R''\text{-}(OH)_a \qquad (IV)$$

$$H_2N\text{-}R'''\text{-}(OH)_a \qquad (V)$$

worin

R, R', R'' und R''', n und a die oben angegebenen Bedeutungen besitzen, in Gegenwart katalytischer Mengen eines stark basischen Katalysators, zweckmässig einer Alkalibase, z.B. eines Alkali-Alkoholats, bei erhöhten Temperaturen bis 200 °C, vorzugsweise 80 bis 160°, umsetzt. Bevorzugt verwendet man als Katalysator ein von dem verwendeten mehrwertigen Alkohol bzw. Aminoalkohol abgeleitetes Alkalimetallalkoholat. Als Ausgangsmaterial (III) verwendet man vorzugsweise die Addukte von Lactamen an aliphatische, cycloaliphatische, aromatische oder heterocyclische Polyisocyanate, ihrer Modifizierungsprodukte oder NCO-Gruppen enthaltenden Voraddukte.

Die neuen Verbindungen eignen sich als Polyole grundsätzlich zur Verwendung in der Polyurethanchemie, etwa als Vernetzer bzw. Kettenverlängerer, wobei es unter Umständen von Interesse sein kann, dass man in das Makromolekül Harnstoffgruppen einbringen kann, ohne Polyamine als Vernetzer verwenden zu müssen. Da ihnen allen ein hoher Schmelzpunkt (> 150 °C bis über 200 °C) gemeinsam ist, sind sie besonders als feinteilige, heterogene Verlängerer in lagerstabilen Einkomponentensystemen von Interesse.

Weiterer Erfindungsgegenstand ist daher die Verwendung der Polyhydroxylverbindungen der Zusammensetzungen (I) bzw. (II) als Reaktionskomponenten zum Aufbau von homogenen oder zelligen Polyurethankunststoffen nach dem Diisocyanatpolyadditionsverfahren, insbesondere als Kettenverlängerer oder Vernetzer beim Aufbau von massiven oder zelligen Polyurethanelastomeren auf Basis von niedrigschmelzenden, höhermolekularen Polyhydroxylverbindungen, Polyisocyanaten und Kettenverlängerungsmitteln in etwa äquivalenten Mengen an NCO zu NCO-reaktiven Gruppen. Besonders interessant ist ihre Verwendung als Reaktionskomponente, dadurch gekennzeichnet, dass man sie in feinteiliger, heterogener Form in Gemischen aus niedrigschmelzenden, höhermolekularen Polyhydroxylverbindungen und Polyisocyanaten oder in den aus den Gemischen gebildeten NCO-Voraddukten, zu lagerstabilen Einkomponenten-Systemen dispergiert und erst durch nachträgliches Erwärmen auf etwa 100 bis 200 °C in die PU-Elastomeren überführt.

Bei der Umsetzung eines mehrfunktionellen Carbamoyllactams vom Typ III mit einem Polyalkohol R''-$(OH)_{a+1}$, etwa einem Glykol, ist grundsätzlich eine Polyaddition möglich, die zu höhermolekularen Produkten führt. Um zu einheitlichen 1:2-Addukten zu gelangen, ist es zweckmässig, einen grösseren Überschuss des Polyalkohols zu verwenden, der dann gleichzeitig als Reaktionsmedium fungiert.

Als Alkoholatanion dient zweckmässig dasjenige des eingesetzten Polyalkohols oder Aminoalkohols, welches man durch Zugabe von etwas Alkalimetall oder einer stark basischen Alkalimetallverbindung, z.B. Alkalihydroxid oder eines Alkalimetallalkoholats anderer Zusammensetzung erzeugt, wobei man den über das Alkoholat eingebrachten Alkohol zweckmässigerweise aus dem sich einstellenden Gleichgewicht abdestilliert. Die Reaktion des Polyalkohols mit dem Carbamoyllactam III gestaltet sich derart, dass man letzteres zu der auf etwa 80 bis 150° erhitzten Lösung des Katalysators im Polyalkohol gibt, worauf es schnell in Lösung geht. Gleichzeitig beginnt das Produkt das erwartete Ureidoalkancarbonsäureesterpolyol (z.B. das Ureidocarbonsäureesterpolyol, sich abzuscheiden. Der Fortgang der Umsetzung kann mit einfachen analytischen Methoden, zweckmassig mit Hilfe der Dünnschichtchromatographie, verfolgt werden. Wenn das Ausgangsmaterial verbraucht ist, neutralisiert man den Ansatz mit einer geeigneten Säure, z.B. einer wässrigen Mineralsaure, und filtriert die Suspension, gegebenenfalls nach Verdünnen mit einem organischen Lösungsmittel (wie Methanol, Ethanol oder Aceton) oder mit Wasser (wenn der verwendete Polyolalkohol wassermischbar ist) ab. Durch Waschen mit Wasser und Lösungsmittel werden Fremdsalze und anhaftender Polyalkohol entfernt, die Produkte sind danach hinreichend rein. Die Mutterlaugen und Waschlösungen können destillativ aufgetrennt und Lösungsmittel sowie nichtumgesetzter Polyalkohol wieder in den Prozess zurückgeführt werden.

Die als Ausgangsmaterialien dienenden polyfunktionellen Carbamoyllactame werden durch Addition von Lactamen an Polyisocyanate gewonnen.

Als Polyisocyanatkomponente sind grundsätzlich alle gebräuchlichen, technisch verfügbaren Polyisocyanate geeignet, z.B. das Toluylendiisocyanat in Form der reinen 2,4- und 2,6-Isomeren und der technisch hergestellten Isomerengemische, ferner das 4,4'-Diisocyanatodiphenylmethan, seine 2,4'- bzw. 2,2'-Stellungsisomeren und seine Isomerengemische und höhermolekularen mehrkernigen Homologen, schliesslich das Hexamethylendiisocyanat, dessen höhermolekulare Modifizierungsprodukte, Biuretgruppen enthaltende Polyisocyanate auf Basis des Hexamethylendiisocyanats, Isophorondiisocyanat, 4,4'-Diisocyanatodicyclohexylmethan, die isomeren Xylylendiisocyanate, α, α, α', α'-Tetramethyl-m/p-Xylylendiisocyanate, oder das 4,4', 4''-Triisocyanatotriphenylmethan. Ebenfalls geeignet sind Isocyanatgruppen enthaltende Voraddukte von Polyisocyanaten, z.B. ein Addukt aus 1 Mol Trimethylolpropan und 3 Mol Toluylendiisocyanat oder ein Addukt aus 1 Mol Dipropylenglykol und 2 Mol Hexamethylendiisocyanat. Weitere geeignete Polyisocyanate werden in DE-OS 2 854 384 und 2 920 501 aufgezählt.

Geeignete, in technischem Massstab verfügbare Lactame sind z.B. Pyrrolidon-2, Piperidon-2, ε-Caprolactam oder Laurinlactam. Bevorzugtes Lactam ist das ε-Caprolactam.

Die verwendeten Polyalkohole IV bzw. Aminoalkohole V sollen bei der Reaktionstemperatur flüssig sein. Als Polyolkomponente sind vor allem niedrige Glykole wie Ethylenglykol und Propylenglykol und deren Oligomere, z.B. Di-, Tri- und Tetra-ethylenglykol bzw. -propylenglykol, 1,3-Propandiol, 1,2-, 1,3-, 1,4- und/oder 2,3-Butandiol oder 1,6-Hexandiol, geeignet.

Die Herstellung der Harnstoffamid-Polyole II erfolgt im wesentlichen gleichartig, nur dass man anstelle eines Glykols oder höherfunktionellen Polyalkohols wie Trimethylolpropan, Glycerin oder Tetriten bzw. Hexiten, einen Monoaminoalkohol oder Monoaminopolyalkohol, z.B. Ethanolamin, Diethanolamin, Propanolamin, Dipropanolamin etc. einsetzt. Amine (ohne Hydroxylgruppen) setzen sich unter den gegebenen Reaktionsbedingungen nicht mit der Carbamoyllactamgruppe von III, auch nicht unter Alkalikatalyse, um. Es reagiert daher bei Verwendung von Alkanolaminen die Hydroxylgruppe des Alkanolamins mit dem Carbamoyllactam III intermediär zu einem Ester mit endständiger Aminogruppe, die sich unter den Reaktionsbedingungen in die stabile Amid-Verbindung vom Typ II umlagert. Man kann daher bei Umsetzung von III (z.B. n = 2) mit Alkanolaminen (z.B. mit einem Monoalkanolamin) etwa äquivalente Mengen (z.B. 2 Mol Monoalkanolamin pro Mol III n = 2) umsetzen, während man bei der Umsetzung von III mit Polyolen vorzugsweise einen Überschuss an Polyolen (in Praxis z.B. bis zu 5 Äquivalente Hydroxylgruppen pro Lactamgruppe) einsetzt, um eine Oligomerenbildung (aus bereits gebildetem I und noch vorhandenem III) zu vermeiden.

Als Lösungs- bzw. Verdünnungsmittel dienen z.B. Dioxan oder besonders zweckmässig Toluol, letzteres obwohl es niedere Aminoalkohole nicht löst. Man suspendiert z.B. den gewählten Aminoalkohol in Toluol, fügt ein Alkalimetallalkoholat oder -hydroxid zu und destilliert im Gleichgewicht freiwerdenden Alkohol bzw. Wasser zusammen mit Toluol ab und fügt bei der vorgesehen Reaktionstemperatur das Lactam-Addukt III hinzu. Das Produkt fällt in feinteiliger, gut filtrierbarer Form an.

Als Katalysatoren dienen z.B. Natrium- oder Kaliumhydroxid, gegebenenfalls in Form alkoholischer Lösungen oder die Natrium- bzw. Kaliumalkoholate, wie z.B. Na- oder K-Methanolat, Ethanolat oder -tert.-Butanolüt. Sie werden in Mengen von 1 bis 10%, vorzugsweise 1 bis 5%, bezogen auf Carbamoyllactamverbindung, eingesetzt.

Die erfindungsgemässen OH-funktionellen Verbindungen I und/oder II können, insbesondere in Kombination mit höhermolekularen (Molekulargewichte 400 bis 6000) niedrigschmelzenden, (< 60 °C) Polyolen, z.B. üblichen Polyether- oder Polyesterpolyolen, mit Polyisocyanaten in bekannter Weise zur Reaktion gebracht werden.

Hierbei werden vorzugsweise die festen Kettenverlängerer I bzw. II in feinteiliger Form, gegebenenfalls nach einem Mahlprozess, mit einer Teilchengrösse von vorzugsweise 1 bis 100 µm, insbesondere 5 bis 50 µm, eingesetzt. Sie liegen somit im Reaktionsgemisch bevorzugt in heterogener Phase vor. Die Topfzeit (Verarbeitungszeit) solcher Systeme richtet sich naturgemäss nach der Reaktivität der in homogener Phase vorliegenden Polyole bzw. Polyisocyanate. Bei Wärmezufuhr erfolgt die Polyaddition dieser Komponenten jedoch zunächst praktisch ohne Mitwirkung der in heterogener Phase vorliegenden OH-funktionellen Verbindungen der Erfindung. Auf diese Weise kann man zu bei Raumtemperatur lagerstabilen PUR-Voraddukten gelangen, die dann zu einem beliebigen Zeitpunkt, gegebenenfalls unter Druck und Formgebung, durch einen Hitzestoss (120 bis 200 °C) verfestigt werden können.

Bevorzugt wird jedoch ein Verfahren nach dem Stand der Technik angewandt, wobei ein Isocyanatgruppen enthaltendes Präpolymer mit den erfindungsgemässen Verbindungen in heterogener Phase zur Reaktion gebracht wird. Hierbei werden bekanntermassen bezüglich Hart- und Weichsegmentverteilung geordnete Endprodukte mit verbesserten mechanischen Eigenschaften erhalten. Die Abmischungen aus den NCO-Präpolymeren und den in feinteiliger Form vorliegenden festen, hochschmelzenden Kettenverlängerern besitzen eine lange Verarbeitungs- bzw. Lagerzeit bei Raumtemperatur, insbesondere in Abwesenheit zusätzlicher Katalysatoren. Der Vorteil solcher Reaktionsmischungen liegt also darin, dass in Folge des hohen Schmelzpunkts und der geringen Löslichkeit der erfindungsgemässen Kettenverlängerer im NCO-Präpolymeren bei Raumtemperatur eine hohe Lagerstabilität solcher Systeme erreicht wird. Diese Langzeit-Reaktiv-Systeme können dann zu einem beliebigen Zeitpunkt, gegebenenfalls nach Zusatz eines Härtungskatalysators, bei erhöhter Temperatur ausgehärtet werden.

Beispiel 1

Herstellung von Hexan-1,6-biscarbamoyl-ε-caprolactam als Ausgangsprodukt

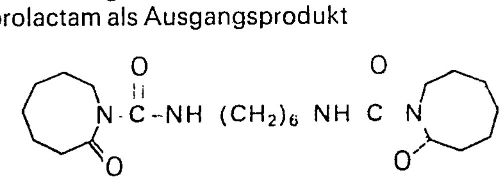

226 g (2 Mol) ε-Caprolactam werden bei 80 °C aufgeschmolzen, im Vakuum kurz entwässert und unter gelegentlicher Kühlung mit 168 g (1 Mol) 1,6-Hexandiisocyanat umgesetzt. Nach dem völligen Verbrauch der NCO-Gruppen (IR-Spektrum, Titration) wurde die Schmelze zum Erstarren auf ein Blech gegossen und zerkleinert. Das Produkt kann aus Toluol/Cyclohexan-Mischungen umkristallisiert werden.

Fp. 84 bis 85 °C
N ber. für $C_{20}H_{34}N_4O_4$ (394): 14,2%
gef. 14,0%

Beispiel 2

Hexan-1,6-bisureidocapronsäure-2-hydroxyethylester (erfindungsgemäss)

$$HOCH_2CH_2O-\overset{O}{\overset{\|}{C}}-(CH_2)_5-NH-\overset{O}{\overset{\|}{C}}-NH-$$

$$(CH_2)_6-NH-\overset{O}{\overset{\|}{C}}-NH-(CH_2)_5-\overset{O}{\overset{\|}{C}}-O-CH_2CH_2OH$$

394 g des Biscarbamoyllactams aus Beispiel 1 werden in 1000 ml (ca. 18 Mol) Ethylenglykol suspendiert. Nach Zusatz von 10 g Na-Methylatlösung (ca. 30%ig in Methanol) wird das Methanol bei 80 °C im Vakuum abdestilliert und das Gemisch anschliessend auf 110 °C erhitzt. Nach dem Verschwinden des Ausgangsmaterials im Dünnschichtchromatogramm (Kieselgel; Chloroform/Methanol 95:5, Anfärbung durch Joddampf) wird der entstandene dichte, feinteilige Niederschlag, dessen Abscheidung durch Verdünnen mit Ethanol vervollständigt werden kann, abgesaugt, mit Ethanol nachgewaschen und getrocknet. Das Produkt lässt sich aus Methanol oder Ethanol umkristallisieren. Die Mutterlaugen und Waschlösungen lassen sich zur Rückgewinnung des nicht umgesetzten Glykols und des Ethanols destillativ auftrennen.

Fp. 155 bis 157 °C
N: ber. für $C_{24}H_{46}N_4O_8$ (518): 10,8%
gef. 10,9%
IR-Spektrum: 1732 $cm^{-1}$ (Ester), 1625 $cm^{-1}$ (aliphatischer, substituierter Harnstoff), 1580 $cm^{-1}$ (NH-Deformation).
Massenspektrum (DCI): Bruchstücke der Massenzahlen 202 und 359, im Einklang mit der Struktur.

Beispiel 3

Hexan-1,6-bisureidocapronsäure -4-hydroxybutylester (erfindungsgemäss)

$$HO\cdot(CH_2)_4\cdot O\cdot\overset{O}{\overset{\|}{C}}\cdot(CH_2)_5NH\cdot\overset{O}{\overset{\|}{C}}\cdot NH(CH_2)_6$$

$$\cdot NH\cdot\overset{O}{C}\cdot NH\cdot(CH_2)_5\cdot CO\cdot O'\cdot(CH_2)_4\cdot OH$$

Die in Beispiel 2 beschriebene Reaktion wurde mit 1,4-Butandiol als Polyolkomponente wiederholt. Das auf die gleiche Weise isolierte Produkt schmilzt bei 143 bis 145 °C.

N: ber. für $C_{28}H_{54}N_4O_8$ (574): 9,76%
gef. 9,8/9,8%
IR-Sepktrum wie Beispiel 2.
Massenspektrum (DCI): Bruchstücke der Massenzahlen 73, 117, 230 und 344, im Einklang mit obiger Struktur.

Beispiel 4

Hexan -1,6-bisureidocapronsäure -6-hydroxyhexylester (erfindungsgemäss)

$$HO-(CH_2)_6-O-\overset{O}{\overset{\|}{C}}-(CH_2)_5-NH-\overset{O}{\overset{\|}{C}}-NH-$$

$$(CH_2)_6-NH-\overset{O}{\overset{\|}{C}}-NH-(CH_2)_5-\overset{O}{\overset{\|}{C}}-O-(CH_2)_6-OH$$

Es wurde wie in Beispiel 2 gearbeitet mit dem Unterschied, dass als Polyol 1,6-Hexandiol verwendet wurde.

Fp.: 135 °C.
N ber. für $C_{32}H_{62}N_4O_8$ (630): 8,9%
gef. 8,9/9,1%
IR- und Massenspektrum (DCI) im Einklang mit der Struktur.

Beispiel 5

4,4'-Diphenylmethanbiscarbamoyl-2-caprolactam (Ausgangsmaterial)

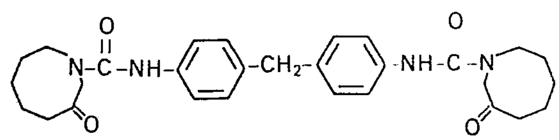

500 g ε-Caprolactam (4,4 Mol) wurden in 400 ml Toluol gelöst, die Lösung wurde durch Andestillieren entwässert, dann wurde bei 80 °C im Laufe von 1 Stunde eine Lösung von 500 g 4,4-Diisocyanatodiphenylmethan, gelöst in 400 ml Toluol, eingetropft. Nach beendeter Reaktion (Verbrauch der NCO-Gruppen) wurde der gebildete Niederschlag abgesaugt, nacheinander mit Toluol und Petrolether gewaschen und getrocknet.

Fp.: 180–182 °C
N ber. für $C_{27}H_{32}N_4O_4$ (476): 11,76%
gef. 11,6/11,7%

Beispiel 6

4,4-Diphenylmethanbisureidocapronsäure-2-hydroxyalkylester (erfindungsgemäss)

$$HO-CH_2CH_2-O-\overset{O}{\overset{\|}{C}}-(CH_2)_5-NH-\overset{O}{C}-NH$$

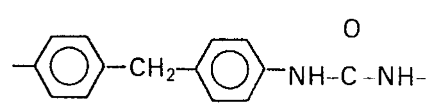

$$(CH_2)_5-\overset{O}{\overset{\|}{C}}-O-CH_2CH_2-OH$$

Wie in Beispiel 2 beschrieben, wurde das Addukt aus Beispiel 5 mit einem Überschuss Ethylenglykol umgesetzt und das Produkt auf die gleiche Weise isoliert.

Fp.: 154 °C
N ber. für $C_{31}H_{44}N_4O_8$ (600): 9,3%
gef. 9,1/9,2%

Beispiel 7

Hexan -1,6-bisureidocapronsäure -2-hydroxyethylamid (erfindungsgemäss)

O O
|| ||
HO–$CH_2CH_2$–NH–C –$(CH_2)_5$–NH–C –NH–

O O
|| ||
$(CH_2)_6$–NH–C –NH–$(CH_2)_5$–C –NH–$CH_2CH_2$–OH

20 g Na-Methylatlösung (30%ig in Methanol) wurden zu 1000 ml trockenem Toluol gegeben und das Methanol zusammen mit etwas Toluol abdestilliert. Dann wurden 394 g (1 Mol) des Lactamaddukts aus Beispiel 1 eingerührt und bei 100 °C 122 g (2 Mol) Ethanolamin im Laufe von 10 Min. zugetropft. Anschliessend wurde 1 Stunde lang bei 100° nachgerührt und der gebildete Niederschlag abgesaugt, mit Ethanol gewaschen und getrocknet. Die Ausbeute ist schon ohne Aufarbeitung der Mutterlauge > 80% der Theorie. Umkristallisiert wird aus Ethanol/Wasser 1:1.

Fp. 204–205 °C
N ber. für $C_{24}H_{48}N_6O_6$ (516): 16,3%
gef. 16,4/16,3%
IR-Spektrum: 1645 $cm^{-1}$, 1618 $cm^{-1}$ (Harnstoff, Amid), 1580 $cm^{-1}$, 1550 $cm^{-1}$ (NH-Deformationsschwingung)

Beispiel 8

4,4'-Diphenylmethanbisureidocapronsäure-2-hydroxyethylamid

O O
|| ||
HO–$CH_2CH_2$–NH–C –$(CH_2)_5$–NH–C –NH–

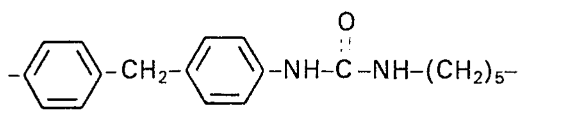

C–NH–$CH_2CH_2$–OH

O

3000 g (49,2 Mol) Ethanolamin wurden mit 15 g Na-Methylatlösung versetzt, das Methanol wurde im Vakuum bei 70 °C abdestilliert. Anschliessend wurden 1500 g (3,15 Mol) des Lactamaddukts aus Beispiel 5 eingerührt, worauf nach 15 Minuten die Abscheidung eines voluminösen Niederschlags einsetzte. Zur Verbesserung der Rührbarkeit wurde mit weiteren 3000 g Ethanolamin verdünnt und die Temperatur auf 100 °C gesteigert. Nach 4 Stunden Nachreaktion wurde abgesaugt und mit Ethanol gewaschen.

Fp. 217 °C
N ber. für $C_{31}H_{46}N_6O_6$ (598): 14,05%
gef. 13,8/14,0%
IR-Spektrum: 1635 $cm^{-1}$ mit Schulter bei 1650 $cm^{-1}$ (Amid, Harnstoff), 1600 $cm^{-1}$ (Aromat), 1560, 1510 $cm^{-1}$ (NH-Deformationsschwingungen).

Beispiele 9 bis 14 (erfindungsgemässe Verwendung zur Herstellung von Polyurethanen)

Beispiel 9

100 g eines NCO-Präpolymeren auf Basis eines linearen Polypropylenglykoletherdiols (Molgewicht 2000, OH-Zahl 56) und 4,4'-Diisocyanatodiphenylmethan (Molverhältnis Polyol zu Diisocyanat wie 1:2), welches einen Isocyanatgruppengehalt von 3,55 Gew.-% aufweist, wurden mit 42,8 g des hochschmelzenden Diols aus Beispiel 2 (Teilchengrösse im Bereich von 20 bis 100 µm) homogen verrührt. Diese Reaktionsmischung besass eine Lagerzeit von mehreren Tagen bei Raumtemperatur (keine Änderung der Viskosität). Erst nach Zugabe von 0,1 g Dibutylzinndilaurat tritt bereits bei Raumtemperatur eine langsame Viskositätssteigerung ein. Durch Erhitzen konnte die Verfestigung des Reaktionsansatzes innerhalb kurzer Zeit vervollständigt werden. So entstanden nach 1 Stunde bei 120–130 °C halbharte zellige Elastomere mit einem Raumgewicht von 0,5 g/$cm^3$.

Beispiel 10

Zu 200 g eines linearen Polyesters aus Adipinsäure, 1,6-Hexandiol und Neopentylglykol (Molverhältnis der Dile 65:35, Molekulargewicht 2000, OH-Zahl 56) wurden bei 60–70 °C 124 g des Diols aus Beispiel 3 gegeben und homogen verrührt. Das Gemisch wurde unter Rühren im Vakuum entgast. Darauf erfolgte langsame Zugabe von 70,1 g 4,4'-Diisocyanatodiphenylmethan in Form einer Schmelze (40–45 °C) und von 0,2 g Blei-II-2-ethylhexanoat, gelöst in Waschbenzin (Octa-Soligen-Pb-24 der Firma Borchers, D-4000, Düsseldorf). Nach ca. 5-minütigem Rühren unter Vakuum wurde das Reaktionsgemisch in eine 20×20×0,5 (Innenmasse in cm) grosse, mit einem Formtrennmittel ausgerüstete Plattenform gegeben. Nach einer Ausheizzeit von 3 Stunden bei 120 °C wurde ein Polyurethanelastomeres mit folgenden Eigenschaften entnommen:

| | |
|---|---|
| Härte (Shore-A): | 83 |
| Zugfestigkeit (MPa): | 13,4 |
| Bruchdehnung (%): | 250 |
| Elastizität (%): | 25 |

Das Formtrennmittel (Siliconöl-Basis) ist Trennmittel V der BAYER-AG, D-5090 Leverkusen.

Beispiel 11

Zu 100 g eines NCO-Präpolymeren auf Basis

eines Polyesters aus Adipinsäure, 1,6-Hexandiol und Neopentylglykol (wie in Beispiel 10) und 2,4-Diisocyanatotoluol (Molverhältnis Diisocyanat zu Polyol wie 2:1, NCO-Gruppengehalt 3,4 Gew.-%) wurden 5 g Diisocyanatodiphenylmethan und 31,1 g des Diols aus Beispiel 2 gegeben. Der Ansatz wurde bei 50 bis 60 °C homogenisiert und unter Vakuum entgast. Danach wurden 0,2 g Blei-II-2-ethylhexanoat zugesetzt. Die resultierende Suspension wurde in einer Plattenform wie in Beispiel 10 beschrieben 3 Stunden lang auf 130 °C erhitzt. Es entstand eine elastische Platte mit einer Härte von 75 bis 80 (Shore A).

Beispiel 12 (erfindungsgemässe Verwendung)

Zu 100 g des in Beispiel 11 beschriebenen NCO-Präpolymeren wurden 5 g 4,4'-Diisocyanatodiphenylmethan und 34,5 g des Diols aus Beispiel 3 in feinverteilter Form gegeben und homogen eingerührt. Die Mischung änderte über mehrere Tage bei Raumtemperatur ihre Viskosität nicht. Die Suspension wurde unter Rühren bei 50 bis 60 °C im Vakuum entgast, mit 0,2 g Blei-II-2-ethylhexanoat versetzt und in einer Form 3 Stunden lang bei 130 °C ausgeheizt. Es entstand ein hochelastischer Formkörper mit einer Härte von 75 Shore A.

Beispiel 13

Die Mischung aus dem in Beispiel 9 beschriebenen NCO-Präpolymer (100 g) und 5 g 4,4'-Diisocyanatodiphenylmethan wurde bei Raumtemperatur mit 38,7 g des Diols aus Beispiel 2 homogen verrührt und die entstandene Suspension bei 50–60 °C im Vakuum unter Rühren entgast. Bei dieser Temperatur erfolgte keine Reaktion, d.h. über mehrere Stunden wurde bei dieser Temperatur kein Viskositätsanstieg beobachtet. Erst nach Zugabe von 0,2 g Blei-II-2-ethylhexanoat setzte langsam die Polyaddition ein. Bei einer Temperatur von 130 °C wurde die Aushärtung in einer Plattenform innerhalb von 3 Stunden vervollständigt. Es entstand eine Platte aus einem Polyurethanelastomeren mit folgenden mechanischen Eigenschaften:

| | |
|---|---|
| Härte (Shore A): | 73 |
| Zugfestigkeit (MPa): | 7,8 |
| Bruchdehnung (%): | 150 |
| Elastizität (%): | 32 |

Beispiel 14

1400 g (0,292 Mol) eines Polyethertriols mit einem Molgewicht von 4800, einer OH-Zahl von 35 und überwiegend primären Hydroxylgruppen, hergestellt durch Addition von Propylenoxid und nachfolgend Ethylenoxid an Trimethylolpropan als Starter, wurden mit 435,9 g (1,744 Mol) 4,4'-Diisocyanatodiphenylmethan zu einem Isocyanatgruppen aufweisenden Polyurethanpräpolymeren umgesetzt. 95,7 Gew.-Teile dieses Präpolymeren wurden mit 54,34 Gew.-Teilen eines Addukts aus 4,4'-Diisocyanatodiphenylmethan und Tripropylengykol (NCO-Gruppengehalt 23 Gew.-%) vermischt. Der NCO-Gruppengehalt dieser Mischung betrug 12,0 Gew.-%. Sie wurde mit 102,5 Gew.-Teilen des Produkts aus Beispiel 5 vermischt und zu einer fliessfähigen Paste homogenisiert. Die Teilchengrösse des festen Diols war kleiner als 100 σm. Nach Zugabe von 0,15 Gew.-Teilen Bleioctoat wurde unter Rühren im Vakuum vollständig entgast. Bei der Lagerung bei Raumtemperatur wurde nach über einem Monat keine Viskositätsänderung festgestellt. 2 cm breite Prüfkörper aus unbehandeltem SMC-Polyesterharz bzw. mit Methylenchlorid entfettetem Eisenblech (Fe) wurden 1 cm überlappend miteinander verklebt und die Verklebungen durch Erhitzen auf 140 °C (15 Minuten) ausgehärtet. Es wurden folgende Scherfestigkeiten gemessen:

| | |
|---|---|
| SMC/SMC | 3,6–3,8 N/mm² |
| Fe/Fe | 2,3–4,0 N/mm² |

## Patentansprüche

1. Harnstoff- sowie Ester- bzw. Amidgruppen aufweisende Polyhydroxylverbindungen der allgemeinen Formeln (I) bzw. (II),

$$R\text{-}[HN\text{-}\overset{O}{\overset{\|}{C}}\text{-}NH\text{-}R'\text{-}\overset{O}{\overset{\|}{C}}\text{-}O\text{-}R''\text{-}(OH)_a]_n \qquad (I)$$

$$R\text{-}[HN\text{-}\overset{O}{\overset{\|}{C}}\text{-}NH\text{-}R'\text{-}\overset{O}{\overset{\|}{C}}\text{-}NH\text{-}R'''(OH)_a]_n \qquad (II)$$

worin

R der Rest eines aliphatischen, cycloaliphatischen, aromatischen oder heterocyclischen, gegebenenfalls modifizierten Polyisocyanats oder eines NCO-Voraddukts, mit der Wertigkeit n,

R' ein $C_3$ bis $C_{11}$-Alkylen,

R'' ein Rest eines mehrwertigen Alkohols der Funktionalität (a+1),

R''' der Rest eines Monoaminoalkohols mit einer Hydroxylfunktionalität a und

a eine ganze Zahl ⩾ 1, und

n 2 bis 6, ist.

2. Polyhydroxylverbindungen der Formeln (I) und (II), nach Anspruch 1, worin

R einen Rest mit der Formel

$-(CH_2)_6-$, $-(CH_2)_6-\underset{CO-NH-(CH_2)_6-}{\underset{|}{N}}-CO-NH-(CH_2)_6-$,

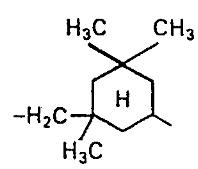 , 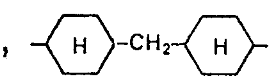 ,

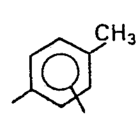 , 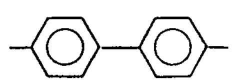 ,

$-R'-NH-CO-O-\underset{CH_3}{\underset{|}{CH}}-CH_2-O-CH_2-\underset{CH_3}{\underset{|}{CH}}-O-CO-NH-R'-$,

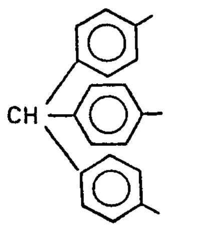

,

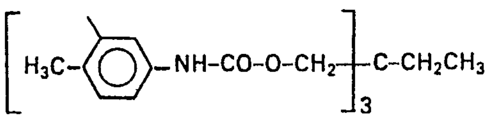

oder

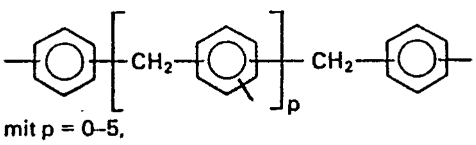

mit p = 0–5,

wobei

R' einen Rest der Formeln

$-(CH_2)_3-$, $-(CH_2)_4-$, $-(CH_2)_5-$ oder $-(CH_2)_{11}-$ Rest;

R'' einen linearen oder verzweigten aliphatischen Rest oder einen cycloaliphatischen Rest der Funktionalität (a+1) und

R''' einen linearen oder verzweigten aliphatischen Rest oder einen cycloaliphatischen Rest der Funktionalität (a+1), bedeutet.

3. Verfahren zur Herstellung der Polyhydroxylverbindungen (I) bzw. (II), entsprechend Anspruch 1 und 2, dadurch gekennzeichnet, dass man Carbamoyllactamgruppen enthaltende Verbindungen der allgemeinen Formel (III)

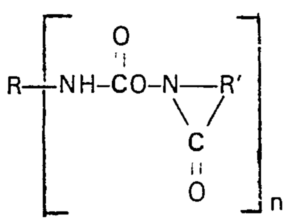

(III)

mit einem mehrwertigen Alkohol der Formel (IV) oder einem Monoaminoalkohol der Formel (V),

$$HO-R''-(OH)_a \qquad (IV)$$

$$H_2N-R'''-(OH)_a \qquad (V)$$

worin

R, R', R'' und R''', n und a die in Anspruch 1 und 2 angegebenen Bedeutungen besitzen,

in Gegenwart katalytischer Mengen stark basischer Katalysatoren aus der Reihe der Alkalibasen, oder Alkali-Alkoholate, bei höheren Temperaturen bis 200 °C, umsetzt.

4. Verfahren nach Anspruch 3, dadurch gekennzeichnet, dass man als Katalysator ein von dem verwendeten mehrwertigen Alkohol (IV) bzw. Aminoalkohol (V) abgeleitetes Alkalimetallalkoholat einsetzt.

5. Verfahren nach Anspruch 3 und 4, dadurch gekennzeichnet, dass man als Ausgangsmaterial (III) die Addukte von Lactamen an aliphatische, cycloaliphatische, aromatische oder heterocyclische Polyisocyanate, ihrer Modifizierungsprodukte oder NCO-Gruppen enthaltenden Voraddukte verwendet.

6. Verwendung der Harnstoff-, Ester- bzw. Amidgruppen aufweisenden Polyhydroxylverbindungen der Zusammensetzungen (I) bzw. (II) nach Ansprüchen 1 und 2, als Reaktionskomponenten zum Aufbau von homogenen oder zelligen Polyurethankunststoffen nach dem Diisocyanatpolyadditionsverfahren.

7. Verwendung als Reaktionskomponente nach Anspruch 6, dadurch gekennzeichnet, dass man sie in feinteiliger, heterogener Form in Gemischen aus niedrigschmelzenden höhermolekularen Polyhydroxylverbindungen und Polyisocyanaten oder in den aus diesen Gemischen gebildeten NCO-Voraddukten, zu lagerstabilen Einkomponenten-Systemen dispergiert und erst durch nachträgliches Erwärmen auf etwa 100 bis 200 °C in die PU-Elastomeren überführt.

## Revendications

1. Composés polyhydroxylés contenant des groupes urée et des groupes ester ou amide, répondant aux formules générales respectives I et II

$$R-[HN-\overset{O}{\overset{\|}{C}}-NH-R'-\overset{O}{\overset{\|}{C}}-O-R''-(OH)_a]_n \qquad (I)$$

$$R-[HN-\overset{O}{\overset{\|}{C}}-NH-R'-\overset{O}{\overset{\|}{C}}-NH-R'''(OH)_a]_n \qquad (II)$$

dans lesquelles

R est le radical d'un polyisocyanate aliphatique, cycloaliphatique, aromatique ou hétérocyclique éventuellement modifié ou d'un préadduct à groupes NCO, de valence n,

R' représente un groupe alkylène en $C_3$–$C_{11}$,

R'' représente le radical d'un alcool polyvalent de fonctionnalité (a+1),

R''' représente le radical d'un monoaminoalcool dont la fonctionnalité en groupes hydroxy est a et

a est un nombre entier supérieur ou égal à 1 et

n est un nombre allant de 2 à 6.

2. Composés polyhydroxylés de formules I et II selon la revendication 1, dans lesquelles R représente un groupe de formule

$$-(CH_2)_6-, \quad -(CH_2)_6-\underset{\underset{CO-NH-(CH_2)_6-}{|}}{N}-CO-NH-(CH_2)_6-,$$

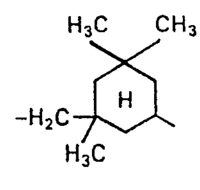

,

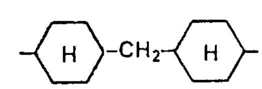

,

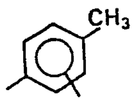 , 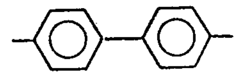 ,

$-R'-NH-CO-O-\underset{CH_3}{CH}-CH_2-O-CH_2-\underset{CH_3}{CH}-O-CO-NH-R'-$,

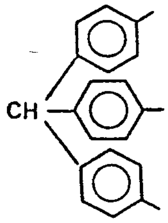 ,

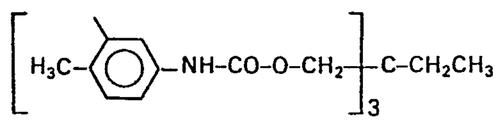

ou

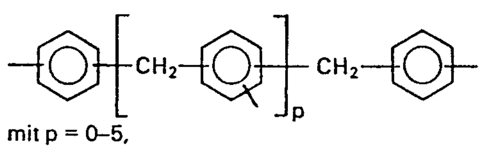

mit p = 0–5,

dans lesquelles

R' représente un groupe de formule

$-(CH_2)_3-$, $-(CH_2)_4$, $-(CH_2)_5-$ ou $-(CH_2)_{11}-$

R'' représente un radical aliphatique linéaire ou ramifié ou un radical cycloaliphatique de fonctionnalité (a+1) et

R''' représente un radical aliphatique linéaire ou ramifié ou un radical cycloaliphatique de fonctionnalité (a+1).

3. Procédé de préparation des composés polyhydroxylés I et II des revendications 1 et 2, caractérisé en ce que l'on fait réagir des composés contenant des groupes carbamoyllactame, répondant à la formule générale III

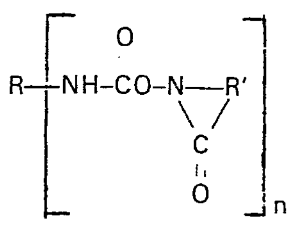 (III)

avec un alcool polyvalent de formule IV ou un monoaminoalcool de formule V respectivement

$$HO-R''-(OH)_a \quad (IV)$$

$$H_2N-R'''-(OH)_a \quad (V)$$

dans lesquelles

R, R', R'' et R''', n et a ont les significations indiquées dans les revendications 1 et 2,

en présence de quantités catalytiques de catalyseurs fortement basiques choisis parmi les bases alcalines ou les alcoolates alcalins, à des températures élevées allant jusqu'à 200 °C.

4. Procédé selon la revendication 3, caractérisé en ce que l'on utilise en tant que catalyseur un alcoolate de métal alcalin dérivé de l'alcool polyvalent IV ou de l'aminoalcool V mis en œuvre.

5. Procédé selon les revendications 3 et 4, caractérisé en ce que l'on utilise en tant que produits de départ III les adducts de lactames sur des polyisocyanates aliphatiques, cycloaliphatiques, aromatiques ou hétérocycliques, leurs produits de modification ou pré-adducts contenant des groupes NCO.

6. Utilisation des composés polyhydroxylés contenant des groupes urée, ester ou amide, de formules respectives I et II selon les revendications 1 et 2 en tant que composants de réaction à la synthèse de résines synthétiques de polyuréthannes homogènes ou alvéolaires par le procédé de polyaddition des isocyanates.

7. Utilisation en tant que composants de réaction selon la revendication 6, caractérisée en ce qu'on les disperse à l'état hétérogène en fines particules, dans des mélanges de composés polyhydroxylés à haut poids moléculaire et à bas point de fusion et de polyisocyanates ou dans les préadducts à groupes NCO formés à partir de ces mélanges, formant ainsi des systèmes à un composant stables à la conservation qui ne sont convertis en les élastomères de polyuréthannes qu'après chauffage à des températures de 100 à 200 °C environ.

## Claims

1. Polyhydroxyl compounds containing urea groups and ester or amide groups and corresponding to the general formula (I) or (II)

$$R-[HN-\overset{O}{\overset{\|}{C}}-NH-R'-\overset{O}{\overset{\|}{C}}-O-R''-(OH)_a]_n \quad (I)$$

$$R-[HN-\overset{O}{\overset{\|}{C}}-NH-R'-\overset{O}{\overset{\|}{C}}-NH-R'''(OH)_a]_n \quad (II)$$

wherein

R denotes the residue of an aliphatic, cycloaliphatic, aromatic or heterocyclic, optionally modified polyisocyanate or of an isocyanate prepolymer having the valency n,

R' denotes a $C_3$ to $C_{11}$-alkylene,

R'' denotes a residue of a polyhydric alcohol having the functionality (a+1),

R''' denotes the residue of a monoamino alcohol having hydroxyl functionality of a and

a denotes an integer ⩾ 1 and

n has a value from 2 to 6.

2. Polyhydroxyl compounds corresponding to formulae (I) and (II) according to claim 1, wherein

R denotes a residue corresponding to one of the following formulae

$-(CH_2)_6-$, 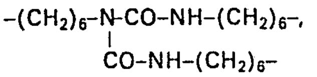

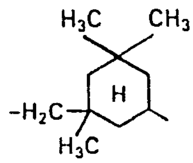 , 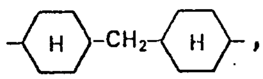 ,

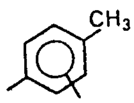 , 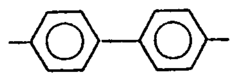 ,

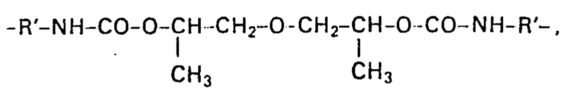

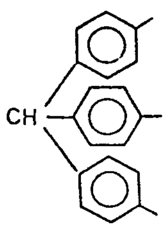 ,

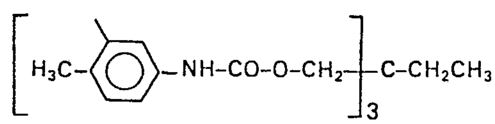

or

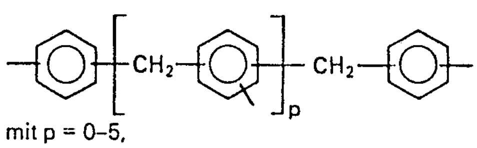

mit p = 0–5,

wherein

p = 0 to 5,

R' denoting a group of one of the following formulae:

$-(CH_2)_3-$, $-(CH_2)_4-$, $-(CH_2)_5-$ or $-(CH_2)_{11}$;

R'' denotes a straight chained or branched aliphatic group or a cycloaliphatic group having the functionality (a+1) and

R''' denotes a straight chained or branched aliphatic group or a cycloaliphatic group having the functionality (a+1).

3. A process for the preparation of the polyhydroxyl compounds (I) and (II) according to claims 1 and 2, characterised in that compounds containing carbamoyl lactam groups and corresponding to the general formula (III)

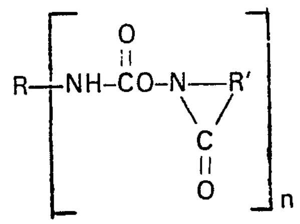 (III)

are reacted with a polyhydric alcohol corresponding to formula (IV) or a monoamino alcohol corresponding to formula (V)

$HO-R''-(OH)_a$ (IV)

$H_2N-R'''-(OH)_a$ (V)

wherein

R, R', R'' and R''', n and a have the meanings indicated in claims 1 and 2,

at elevated temperatures of up to 200 °C in the presence of catalytic quantities of strong basic catalysts from the series of alkali metal bases or alkali metal alcoholates.

4. A process according to claim 3, characterised in that the catalyst used is an alkali metal alcohol derived from polyvalent alcohol (IV) or amino alcohol (V) used.

5. A process according to claims 3 and 4, characterised in that the products of addition of lactams to aliphatic, cycloaliphatic, aromatic or heterocyclic polyisocyanates, their modification products or prepolymers containing isocyanate groups are used as starting material (III).

6. The use of polyhydroxyl compounds containing urea and ester or amide groups and having the compositions (I) and (II) according to claims 1 and 2 as reactants for the synthesis of homogeneous or cellular polyurethane plastics by the diisocyanate polyaddition process.

7. Their use as reaction components according to claim 6, characterised in that they are dispersed in a finely divided, heterogeneous form in mixtures of low melting, relatively high molecular weight polyhydroxyl compounds and polyisocyanates or in isocyanate prepolymers formed from these mixtures to form storage stable one-component systems and are not converted into PU elastomers until subsequently heated to about 100 to 200 °C.